# EUROPEAN PATENT APPLICATION

(11) **EP 2 127 683 A1**
(43) Date of publication of application: **02.12.2009**
(21) Application number: 08157163.0
(22) Date of filing: 29.05.2008
(51) Int. Cl.: A61K 51/10, A61K 49/16, A61K 101/00, A61K 101/02, A61K 103/00, A61K 103/10, A61K 103/20, A61K 103/32

(54) **Anti-Met monoclonal antibody, fragments and derivatives thereof for use in tumor imaging, corresponding compositions and kits**

(71) Applicant: Metheresis Translational Research SA, 6900 Lugano (CH)
(72) Inventor: Comoglio, Paolo Maria, 10133 Torino (IT); Carminati, Paolo, 20121 Milano (IT); Van Dongen, Guus c/o Dept. of Otolaryngology, Head and Neck Surgery, VU University Medical Center Amsterdam (NL)
(74) Representative: Freyria Fava, Cristina

(57) **Abstract**

An immuno-imaging agent for the detection of tumor cells by means of an immuno-imaging technique, including at least one of:
- an anti-Met monoclonal antibody,
- a fragment of an anti-Met monoclonal antibody containing the epitope binding region thereof,
- a genetically engineered antibody containing the epitope binding region of an anti-Met monoclonal antibody,
- a humanized antibody containing the epitope binding region of an anti-Met monoclonal antibody, or combinations thereof,

wherein the anti-Met monoclonal antibody is produced by the hybridoma cell line ICLC PD 05006, and corresponding compositions and kits.

## Description

### Field of the invention

The present invention concerns the use of a monoclonal antibody, fragments and/or derivatives thereof, as diagnostic tool for the detection of neoplastic cells. In particular, the present invention concerns the use of an anti-Met monoclonal antibody, fragments and/or derivatives thereof directed against the extracellular domain of hepatocyte growth factor receptor as a diagnostic tool for the detection of tumorigenic cells.

### Background of invention

The *MET* oncogene, encoding the tyrosine kinase receptor for Hepatocyte Growth Factor (HGF), controls genetic programs leading to cell growth, invasion and protection from apoptosis. Deregulated activation of HGFR is critical not only for the acquisition of tumorigenic properties but also for the achievement of the invasive phenotype. The role of *MET* in human tumors emerged from several experimental approaches and was unequivocally proved by the discovery of *MET* activating mutations in inherited forms of carcinomas. Moreover, *MET* constitutive activation is frequent also in sporadic cancers and laboratories studies have shown that the *MET* oncogene is overexpressed in tumors of specific histotypes or is activated through autocrine mechanisms. Besides, the prevalence of abnormal MET expression is typically higher in metastases than in primary tumors, and is associated with poor clinical prognosis. As an example, the *MET* gene is amplified in hematogenous metastases of colorectal carcinomas.

What is more, Engelman et al. (Science 2007; 316:1039-43) recently showed that lung tumours can develop resistance to epidermal growth factor receptor (EGFR) inhibitors as a result of amplification of the *MET* oncogene, while inhibition of Met signalling restored their sensitivity to EGFR inhibitors. This makes Met, on the analogy of e.g. EGFR, an interesting target for tumour detection, cancer prognostication, and anti-cancer therapy, even in the absence of genetic alterations.

Monoclonal antibodies (MAbs) are particularly attractive for this purpose. Especially intact MAbs (150 kDa) have shown value, because their long residence time allows neutralization/blockage of growth factors or growth factor receptors for a prolonged period of time. Neutralizing anti-HGF MAbs have been used, but their application is limited to tumours with HGF-dependent Met activation. Recently, it emerged that probably the best way to block the HGF/Met-induced invasive program is the competition with the Met receptor itself.

Recently, the group of Vande Woude pioneered gamma camera imaging for visualization of Met expressing tumours as disclosed in Hay RV, et al., Clin Cancer Res 2005; 11:7064s-9s and WO-A-2003/057155. For this purpose the anti-MET MAbs (MetSeek™) designated Met3 and Met5 were used. Antibodies were labelled with ¹²⁵I to enable gamma-camera imaging. Using relatively large tumours localized in the right thigh of the mice, i.e. far outside the abdominal region where ¹²⁵I uptake was high, Met5 gave better tumour visualization and retention than Met3. The authors, nevertheless, suggest to employ a mixture of MAbs recognizing different epitopes of Met to improve the diagnostic results. Moreover, the antibodies disclosed in WO-A-2003/057155 do not allow a good detection of tumor cells expressing low levels of MET and tumours at early stage of development (i.e. having small masses) or new metastasis, especially if localized in the abdominal region.

### Summary of the invention

The need is therefore felt for improved solutions enabling as early as possible reliable detection of tumorigenic cells expressing MET, the tyrosine kinase receptor for Hepatocyte Growth Factor.

The object of this disclosure is providing such improved solutions.

According to the invention, the above object is achieved thanks to the subject matter recalled specifically in the ensuing claims, which are understood as forming an integral part of this disclosure.

The invention provides the use of a monoclonal antibody, fragments and/or derivatives thereof directed against the extracellular domain of Hepatocyte Growth Factor Receptor (HGFR) as a diagnostic reagent to detect neoplastic cells, wherein the monoclonal antibody allows for the detection of such neoplastic cells even when MET is expressed at very low level on the cellular surface.

An embodiment of the invention provides an immuno-imaging agent for the detection of tumor cells by means of an *in vivo* immuno-imaging technique, the immuno-imaging agent including at least one of:
- an anti-Met monoclonal antibody,
- a fragment of an anti-Met monoclonal antibody containing the epitope binding region thereof,
- a genetically engineered antibody containing the epitope binding region or the complementary determining regions (CDRs) of an anti-Met monoclonal antibody,
- a humanized antibody containing the epitope binding region or the complementary determining regions (CDRs) of an anti-Met monoclonal antibody, or combinations thereof,
wherein the anti-Met monoclonal antibody - named DN30 - is produced by the hybridoma cell line deposited by Advanced Biotechnology Center (ABC), Interlab Cell Line Collection (ICLC), S.S. Banca Cellule e Colture in GMP, Largo Rosanna Benzi 10, Genova, Italy with accession number ICLC PD 05006.

An embodiment of the invention provides DN30 monoclonal antibody, its fragments or genetically engineered or humanized antibodies containing DN30 epitope binding region or CDRs coupled to a detectable signaling moiety, which is suitable for use in gamma camera imaging technique, MRI technique or PET technique.

A further embodiment of the instant invention concerns a diagnostic composition comprising DN30 monoclonal antibody, its fragments or genetically engineered or humanized antibodies containing DN30 epitope binding region or CDRs as the immuno-imaging agent, wherein the detection of said immuno-imaging agent occurs by means of *in vivo* immuno-imaging techniques, such as gamma camera imaging technique/SPECT, MRI technique or PET technique.

In a still further embodiment, the present invention provides a diagnostic kit including a first vial containing a diagnostic composition comprising DN30 monoclonal antibody, its fragments or genetically engineered or humanized antibodies containing DN30 epitope binding region or CDRs as an immuno-imaging agent and optionally a second vial containing a detectable signaling moiety to be coupled to the immuno-imaging agent.

In an embodiment, the immuno-imaging agent is coupled either directly (e.g. via tyrosine residues of the antibody when ¹²⁴I is used) or indirectly (e.g. via a linker - as a metal chelating agent) to a detectable signaling moiety. In another embodiment, the immuno-imaging agent is coupled to a molecule able to be coupled (either *in vitro* or *in vivo*) to the detectable signaling moiety at the time and place of use.

The detectable signaling moiety may be already active or activatable, wherein in such a case the detectable signaling moiety is activatable i.a. by substitution with an active element, e.g. a metal, a radionuclide or a positron emitter suitable to be detected.

The detectable signaling moiety is selected as a function of the immuno-imaging technique employed for the diagnosis, i.e. gamma-emitting radionuclide (or gamma-emitter) in case of gamma camera-imaging technique/SPECT, metal or positron emitter in case of MRI or PET imaging techniques, respectively.

The present invention allows the detection of MET expression on the surface of the cells themselves, in a tissue, in an organ or in a biological sample, i.e. either *in vitro* or *in vivo,* for the purpose of diagnosis, prognosis and/or post-therapy monitoring.

The use of DN30 as immuno-imaging agent allows early detection of tumorigenic cells expressing Met on their surface even if MET expression on the surface of these cells is very low because of the high affinity (2.64 x 10e-9) of DN30 for Met. Further advantages in using DN30 as immuno-imaging agent lie in its unexpected property of being adherent to the tumorigenic cell surface for a quite long period of time. Moreover, DN30 being able to be internalized within the tumorigenic cells allows to achieve an unexpectedly good tumour-to-nontumour ratio and consequently a surprising sensibility and specificity for the detection of tumorigenic cells.

### Detailed description of the invention

The present invention will now be described in detail in relation to some preferred embodiments by way of non-limiting examples with reference to the annexed drawings, wherein:
- **Figure 1** shows immunohistochemical staining of Met expression with biotinylated DN30 on cytospins of GTL-16 (a) and FaDu (b) cells, and on frozen sections of GTL-16 (c) and FaDu (d) xenografts.
- **Figure 2** shows sequential HRRT PET images (coronal slices) of two different GTL-16 xenograft-bearing nude mice at 1, 2, 3, and 4 days after i.v. injection with ⁸⁹Zr-DN30 (2.6 MBq, 100 µg MAb). Image planes have been chosen where both the left and right tumours are visible. Xenografts are indicated by arrows.
- **Figure 3** shows sequential HRRT PET images (coronal slices) of two different FaDu xenograft-bearing nude mice at 1, 2, 3, and 4 days after injection with ⁸⁹Zr-DN30 (1.8 MBq, 100 µg MAb). Image planes have been chosen where both the left and right tumours are visible. Xenografts are indicated by arrows.
- **Figure 4** represents the nucleic acid (a) and aminoacid (b) sequence of DN30 heavy chain. The CDR regions are underlined both in the nucleotide and aminoacid sequence.
- **Figure 5** represents the nucleic acid (a) and aminoacid (b) sequence of DN30 light chain. The CDR regions are underlined both in the nucleotide and aminoacid sequence.

For optimal application of anti-Met MAbs in molecular imaging procedures the anti-Met MAbs should be capable of being internalized in tumor cells, as the case of DN30 MAb, after binding to the tumour cells and should have a high affinity for Met, i.e. able to bind Met even if the expression levels of Met on the cell surface are very low. Nevertheless, in case of non-internalizing MAbs, optimal molecular imaging can be obtained selecting suitable detectable signaling moieties as e.g. ¹²⁴I for PET imaging.

Traditionally for imaging of radioactivity, planar imaging with a gamma-camera or single photon emission computerized tomography (SPECT) have been used. More recently, positron emission tomography (PET) emerged as an attractive option for *in vivo* imaging of MAbs (immuno-PET). PET offers a high resolution and sensitivity combined with the unique ability to measure tissue concentrations of radioactivity in three dimensions.

The imaging procedures cited in the foregoing are per se conventional in the art and do not require to be further detailed herein.

To enable immuno-imaging of MAbs with the aforementioned immuno-imaging techniques appropriate detectable signaling moieties - with a half-life compatible with the time needed to achieve optimal tumor-to-nontumour ratios - has to be securely coupled (directly or through suitable chelating molecules) to the immuno-imaging agent. This strategy can be followed when the labelled immuno-imaging agent can be delivered to the research/hospital site of use within 1-2 days. This is the advantage of long-lived positron emitters with a half life time of 3-4 days. If delivering takes about 30 hours, then it is necessary to deliver the labelled immuno-imaging agent with 30% more radioactivity, being the quality of the conjugate well preserved up to 2 days.

In case where the delivery of the immuno-imaging agent takes more than 30 hours other labeling procedures have to be followed. As an example, the immuno-imaging agent may be delivered in a first vial as a conjugate to a suitable bifunctional chelating molecule (first conjugate) and the detectable signaling moiety may be delivered separately in a second vial. The detectable signaling moiety is ready to be coupled to the immuno-imaging agent through the bifunctional chelating molecule. In such a case labeling can be easily performed at the research/hospital site of use. The first conjugate is labeled at room temperature with the detectable signaling moiety at the time needed.

In the following, attention will be paid to PET imaging technique, only as an exemplary embodiment of the invention.

In the present disclosure, the potential of MAb DN30 for quantitative PET imaging of Met expressing human tumour xenografts has been disclosed.

Visualization and quantification of MAb biodistribution using PET requires a suitable positron-emitting radionuclide. Two positron emitters seem well suited for imaging of intact MAbs, ⁸⁹Zr (t_{1/2}=78.4 h) and ¹²⁴I (t_{1/2}=100.3 h), since the physical half-life of these radionuclides matches the time needed for MAbs to achieve optimal tumour-to-nontumour ratios (2-4 days for intact MAbs). Copper-64 (t_{1/2}=12.7 h), yttrium-86 (t_{1/2}=14.7 h) and bromine-76 (t_{1/2}=16.2 h) are also used for this purpose, but are less optimal for imaging at later time points.

In previous studies, the present inventors described procedures for production and purification of large amounts of these positron emitters and for their stable coupling to MAbs, with maintenance of the *in vivo* biodistribution characteristics of the latter (see Verel I, et al., Eur J Nucl Med Mol Imaging 2004; 31:1645-52 and Verel I, et al., J Nucl Med 2003; 44:1271-81). While ⁸⁹Zr is coupled via a chelate to the lysine residues of a MAb, ¹²⁴I can be coupled directly via tyrosine residues. Moreover, the present inventors demonstrated that ⁸⁹Zr is particularly suitable for PET imaging of internalizing MAbs, and ¹²⁴I for non-internalizing MAbs. In contrast to directly labelled ¹²⁴I, ⁸⁹Zr is trapped in the cell after internalization of the MAb (residualization). Residualization also occurs to some extent in organs of MAb catabolism like liver, kidney and spleen. The clinical potential of ⁸⁹Zr-immuno-PET and PET-CT for tumour detection was recently demonstrated also in head and neck cancer patients (Borjesson et al., Clin Cancer Res 2006;12: 3133-40).

The present inventors described also an alternative procedure for labelling an immuno-imaging agent with a detectable signaling moiety, which allows for storing the immuno-imaging agent suitably modified but not still radiolabelled for a quite long period of time. The labelling may be performed immediately before use. In such a case, the immuno-imaging agent is previously coupled to p-isothiocyanatibenzyl-desferrioxamine, which allows for subsequent coupling to the positron-emitter, e.g. ⁸⁹Zr or ⁶⁸Ga. The premodified immuno-imaging agent can be stored at -20°C until the day of planned use.

In this disclosure, biodistribution and PET imaging studies were described in nude mice using two different xenograft lines with divergent levels of Met expression, the human gastric carcinoma cell line GTL-16 with high expression and the HNSCC cell line FaDu with low expression. The FaDu cell line was also chosen as a challenging model to examine imaging quality of radiolabelled DN30.

Both long-lived positron emitters, the residualizing radionuclide ⁸⁹Zr and the nonresidualizing radionuclide ¹²⁴I, were considered as candidates for PET imaging with DN30. If a MAb is internalized after binding to the tumour cell, the use of residualizing radionuclide for imaging might be advantageous because of higher tumour-to-nontumour ratios. The biodistribution of coinjected ⁸⁹Zr-DN30 and ¹³¹I-DN30 (¹³¹I as a substitute for ¹²⁴I to facilitate simultaneous counting) in GTL-16 xenograft bearing mice indeed revealed major differences in tumour uptake. Tumour uptake was substantially higher for ⁸⁹Zr compared to ¹³¹I at all time points, and almost four times higher at the latest time point (5 days p.i.). As a result, tumour-to-nontumour ratios were significantly better for ⁸⁹Zr-DN30. In contrast to ⁸⁹Zr, levels of ¹³¹I in tumours never exceeded levels of ¹³¹I in blood.

On the basis of these results, the inventors consider that the residualizing radionuclide ⁸⁹Zr is better suited for PET imaging with DN30 than nonresidualizing iodine (¹²⁴I). Although, indirect radio-iodination methodologies can be applied that will result in higher retention of radioactivity in tumour cells after the internalization of labelled MAbs.

Using PET with ⁸⁹Zr-DN30, GTL-16 tumours as small as 11 mg could be clearly visualized from day 1 p.i. onwards. Also tumours with low Met expression, like the FaDu xenografts, could be clearly delineated with ⁸⁹Zr-DN30 immuno-PET. Moreover, the potential of ⁸⁹Zr-immuno-PET for non-invasive quantification of DN30 biodistribution was illustrated by the excellent correlation between PET-assessed tumour uptake data and ex vivo tumour uptake data (R²=0.98). In clinical trials, quantitative PET imaging would be preferable over repeated tumour biopsies, especially because tumours are often heterogeneous (resulting in non-representative biopsies) and difficult accessible.

Despite clear visualization of small tumours, ⁸⁹Zr-DN30 showed relatively high uptake in liver and spleen, especially when administered at relatively low protein dose (Table 2). Part of the liver and spleen uptake is due to residualization of ⁸⁹Zr after catabolism of the conjugate in these organs, as was also observed in inventors' previous studies with ⁸⁹Zr-cetuximab (Erbitux) and ⁸⁹Zr-ibritumomab tiuxetan (Zevalin) in the same animal model. Nevertheless, we hypothesize that the enhanced uptake of radioactivity in liver and spleen might be partly an artefact related to the nude mouse model, which will not occur in humans. At this point, even better images can be expected in clinical studies.

DN30 is a murine MAb of the IgG₂ₐ isotype. Sharkey et al. (Cancer Res 1991; 51:3102-7) and Van Gog et al. (Cancer Immunol Immunother 1997; 44:103-11) described the phenomenon of fast blood clearance of murine MAbs with concomitant high accumulation in liver and spleen, in various strains of outbred *nu*/*nu* mice. Fast blood clearance and enhanced liver and spleen uptake did especially occur when murine IgG₂ₐ or IgG_{2b} isotype MAbs, young animals, or a low MAb dose were used. The phenomenon was most prominent in animals with low endogenous IgG titers. The authors postulated that rapid removal of MAb from the blood might be mediated by Fc-binding receptors in e.g. liver and spleen as long as endogenous MAb titres are low. A very similar phenomenon was observed in this disclosure when the MAb dose of DN30 was varied: lower MAb dose was associated with enhanced and variable blood clearance and a concomitant increased uptake in liver and particularly spleen (Table 2).

As far as the radiochemistry concerns, taking the step to clinical evaluation of immuno-PET with ⁸⁹Zr-labeled humanized or fully human anti-Met MAbs is relatively straightforward. Using the same radiochemical approach, the inventors recently reported on an immuno-PET study with ⁸⁹Zr-labeled anti-CD44v6 MAb U36 (75 MBq) for detection of lymph node metastases in 20 head and neck cancer patients and with ⁸⁹Zr-ibritumomab tiuxetan for prediction of ⁹⁰Y-ibritumomab tiuxetan biodistribution with PET in non-Hodgkin's lymphoma patients. With both ⁸⁹Zr-conjugates, excellent PET images were obtained.

DN30 pursuant to the present invention can be produced by conventional methods in animals or, preferably, by genetic engineering techniques.

The use of the monoclonal antibody DN30 according to the present invention is intended to include also the use of genetically engineered and humanized antibodies. Genetically engineered and humanized antibodies and methods for their production are known in the art. See, for a review Clark M. Imm. Today, 2000; 21:397-402.

The use of DN30 also include the use of fragments containing the epitope binding region or Complementary Determining Regions (CDRs) thereof as Fv, scFv, Fab, Fab', F(ab')₂ fragments. Conventional fragments are typically produced by proteolitic cleavage, but can also be produced by chemical synthesis, such as liquid or solid phase synthesis, as well as by recombinant DNA techniques. With the expression "epitope binding region" is meant the portion of the antibody recognizing the antigen, i.e. the antigen-binding site. With the expression "Complementary Determining Region" is meant the short aminoacid sequence found in the variable domains of the antibody that complements the antigen and therefore provides the antibody with its specificity for that particular antigen.

### MATERIAL AND METHODS

### DN30 nucleotide and aminoacid sequences

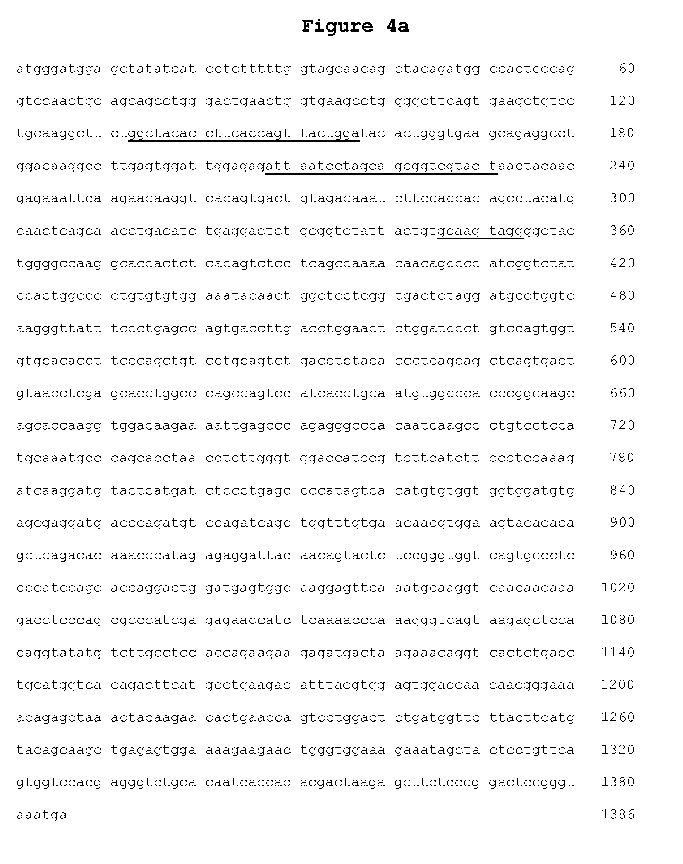

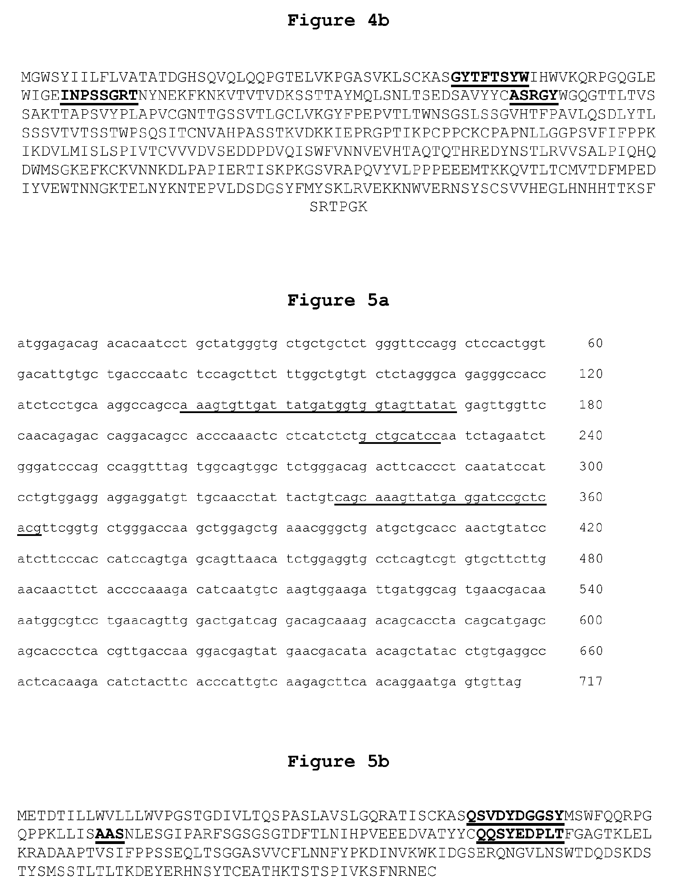

The translation of the DN30 heavy chain nucleotide sequence corresponding to the SEQ ID No.: 1 and figure 4a is reported in figure 4b and SEQ ID No:6.

The nucleotidic and aminoacid sequences corresponding to the CDR regions are underlined in Figures 4a and 4b; their aminoacid sequences are: CDR-H1: GYTFTSYW (SEQ ID NO.:8); CDR-H2: INPSSGRT(SEQ ID NO.:9); CDR-H3: ASRGY (SEQ ID NO.:10).

The translation of the DN30 light chain nucleotide sequence corresponding to the SEQ ID No.: 2 and figure 5a is reported in figure 5b and in SEQ ID NO.:7.

The nucleotidic and aminoacid sequences corresponding to the CDR regions are underlined in Figures 5a and 5b; their aminoacid sequences are: CDR-L1: QSVDYDGGSY (SEQ ID NO.:11); CDR-L2: AAS (SEQ ID NO.:12); CDR-L3: QQSYEDPLT (SEQ ID NO.:13).

### Monoclonal antibodies, cell lines, and radioactivity

The murine IgG₂ₐ MAb DN30 (7.0 mg/mL), directed against the extracellular domain of Met (K_{d} of 2.64 x 10⁻⁹ M), was obtained from the Institute for Cancer Research and Treatment (IRCC), University of Turin Medical School, Italy. The hybridoma cell line was deposited by Advanced Biotechnology Center (ABC), Interlab Cell Line Collection (ICLC) Italy, with accession number ICLC PD 05006. Selection, construction, and production of DN30 have been described in Prat M, et al., J Cell Sci 1998; 111:237-47.

The human gastric carcinoma cell line GTL-16, in which the *MET* proto-oncogene is amplified and overexpressed (Ponzetto C, et al., Oncogene 1991; 6:553-9), was obtained from IRCC and deposited on April 16, 2008 by Advanced Biotechnology Center (ABC), Interlab Cell Line Collection (ICLC) Italy, with accession number ICLC PD 08003. The head and neck squamous cell carcinoma (HNSCC) cell line FaDu was obtained from Karl-Heinz Heider (Boehringer Ingelheim, Vienna, Austria) (Rangan SRS, Cancer 1972; 29:117-21).

⁸⁹Zr (2.7 GBq/mL in 1 M oxalic acid) was produced by BV Cyclotron (Amsterdam, The Netherlands) by a (p,n) reaction on natural yttrium-89 (⁸⁹Y) and isolated with a hydroxamate column (Verel I, et al., J Nucl Med 2003; 44:1271-81). ¹³¹I (7.4 GBq/mL in 0.01 M sodium hydroxide) was purchased from GE Healthcare Life Sciences (Uppsala, Sweden).

### Immunohistochemical staining of cell lines and xenografts

The cell lines GTL-16 and FaDu were characterized for Met expression by performing immunocytochemistry with biotinylated DN30. In short, cells were trypsinized, spun onto glass slides at a density of 5 x 10⁴ cells/spin, and the glass slides were air dried overnight. After fixing the cells with freshly prepared 2% paraformaldehyde for 10 min, the slides were incubated in Tris buffer (50 mM, pH 7.2) containing 2% bovine serum albumin (BSA) for 30 min, followed by incubation with biotinylated DN30 for 1 h at room temperature. After extensive washing with the Tris buffer, the cells were incubated with streptavidin-alkaline phosphatase (ChemMate Detection Kit; Dako, Glostrup, Denmark) for 1 h at room temperature. Colour developing was performed using freshly prepared substrate from the kit, followed by washing with demineralized water.

In addition, immunohistochemistry was performed on frozen sections of GTL-16 and FaDu xenografts. Cryostat sections (5 µm) were air dried and fixed in 2% paraformaldehyde for 10 min. Met staining was performed as described above.

### Radiolabelling

a) For radiolabelling of DN30 with ⁸⁹Zr, bifunctional metal-chelating moiety had to be conjugated to the MAb as described previously by Verel et al. J Nucl Med 2003; 44:1271-81. Briefly, the chelate desferal (Df; Novartis, Basel, Switserland) was succinylated (*N*-sucDf), temporarily filled with stable iron [Fe(III)], and coupled to the lysine residues of DN30 by means of a tetrafluorophenol-*N*-sucDf ester. After removal of Fe(III) by transchelation to EDTA, the premodified MAb was purified on a PD10 column (GE Healthcare Life Sciences). Approximately 1 *N*-sucDf moiety was coupled per DN30 molecule assessed by using ⁵⁹Fe (Perk LR, et al., Eur J Nucl Med Mol Imaging 2006; 33:1337-45). Subsequently, *N*-sucDf-DN30 (0.5 mg) was labelled with ⁸⁹Zr (max. 37 MBq) in 0.5 M HEPES buffer at pH 7.0. Finally, ⁸⁹Zr-*N*-sucDf-DN30 was purified on a PD10 column (eluent: 0.9% sodium chloride/gentisic acid 5 mg/mL, pH 5.0). ⁸⁹Zr-*N*-sucDf-DN30 will be abbreviated to ⁸⁹Zr-DN30 in the rest of this article.
   Radioiodination of DN30 with ¹³¹I was performed essentially as described in Visser GW et al., J Nucl Med 2001; 42:509-19. ¹³¹I was used as substitute of ¹²⁴I to facilitate dual isotope counting together with ⁸⁹Zr in the biodistribution studies (see later). In short, to a 20 mL β-scintillation glass vial coated with 75 µg IODO-GEN (Pierce Biotechnology, Rockford, IL), 0.05 mL of 0.5 M sodium phosphate (pH 7.4), 50-200 µg DN30 in 0.45 mL of 0.1 M sodium phosphate (pH 6.8), and 9-18.5 MBq of ¹³¹I were added, successively. After gentle shaking for 4 min at room temperature, the reaction was quenched by the addition of 0.1 mL of 25 mg/mL ascorbic acid (pH 5.0). Finally, ¹³¹I-DN30 was separated from non-reacted ¹³¹I by purification on a PD10 column (eluent: 0.9% sodium chloride/ascorbic acid 5 mg/mL, pH 5.0).
b) Radiolabelling of DN30 with ⁸⁹Zr can also be performed according to a second protocol which allows storing of DN30 suitably modified but not still radiolabelled until the day of planned use.

In a solution of DN30 at a concentration equal or higher than 2 mg ml⁻¹ having pH=8.9-9.1, p-isothiocyanatibenzyl-desferrioxamine in DMSO was dissolved at a concentration of between 2 and 5 mM (1.5-3.8 mg ml⁻¹) and mixed immediately, keeping the DMSO concentration below 5% in the conjugation reaction mixture. p-isothiocyanatibenzyl-desferrioxamine had to be in a 3-fold molar excess over the molar amount of DN30. The reaction mixture was incubated for 30 min at 37°C. The Df-DN30 conjugate was then purified using a PD-10 column according to the following protocol: i) the PD10 column was rinsed with 20 ml 0.9% NaCl/gentisic acid 5 mg ml⁻¹ (pH = 4.9-5.3); ii) the conjugation reaction mixture was pipetted onto the column and the flow-through discarded; iii) 1.5 ml 0.9% NaCl/gentisic acid 5 mg ml⁻¹ (pH = 4.9-5.3) was pipetted onto the column and the flow-through discarded and iv) 2 ml 0.9% NaCl/gentisic acid 5 mg ml⁻¹ (pH = 4.9-5.3) was pipetted onto the PD-10 column and the Df-DN30 conjugate collected.

After that, the Df-DN30 conjugate can be stored at -20°C until the day of planned use. The Df-DN30 conjugate is stable in storage for at least several weeks.

The Df-DN30 conjugate could then be labelled (when necessary for use) with ⁸⁹Zr according to the following protocol: i) between 25-200 µl (A) of ⁸⁹Zr oxalic acid solution (between 37 and 185 MBq) was pipetted into a glass "reaction vial"; ii) while gently shaking, 200 - A µl 1M oxalic acid was added into the reaction vial. Subsequently, 90 µl 2 M Na₂CO₃ were pipetted into the reaction vial and incubated for 3 minutes at room temperature; iii) while gently shaking, successively 0.30 ml 0.5 M HEPES (pH=7.2), 0.71 ml of Df-DN30 (typically 1-3 mg), and 0.70 ml 0.5 M HEPES (pH=7.2) were pipetted into the reaction vial, keeping the pH of the labeling reaction in the range of 6.8-7.2. iv) The reaction mixture was then incubated for 1 h at room temperature while gently shaking. v) Meanwhile, a PD10 column was rinsed with 20 ml 0.9% NaCl/gentisic acid 5 mg ml⁻¹ (pH=4.9-5.3); vi) after 1 h incubation, the reaction mixture was pipetted onto the column and the flow-through discarded. vii) 1.5 ml 0.9% NaCl/gentisic acid 5 mg ml⁻¹ (pH = 4.9-5.3) was pipetted onto the column and the flow-through discarded; viii) 2 ml 0.9% NaCl/gentisic acid 5 mg ml⁻¹ (pH=4.9-5.3) was pipetted to the PD-10 column and the purified radiolabeled DN30 collected. ix) The purified radiolabeled DN30 was analyzed by ITLC and HPLC. When the radiochemical purity was greater than 95%, the solution was ready for storage at 4 °C or dilution in 0.9% NaCl/gentisic acid 5 mg ml⁻¹ (pH=4.9-5.3) for *in vitro* or *in vivo* studies. The radiolabeled protein is stable in storage for at least several days.

Gentisic acid was introduced during labeling and storage to prevent deterioration of the protein integrity by radiation. Typically, 0.9-1.5 Df moieties were coupled per DN30 antibody. Radiolabeling of the Df-conjugated mAb with ⁸⁹Zr resulted in overall labeling yields of >85%. Resulting ⁸⁹Zr-mAb conjugates were optimal with respect to radiochemical purity (>95% according to ITLC and analytical HPLC), immunoreactivity, and in vivo stability.

### Analyses

After each preparation of ⁸⁹Zr-DN30 or ¹³¹I-DN30, the conjugates were analysed by instant thin-layer chromatography (ITLC) for radiolabelling efficiency and radiochemical purity, and by high performance liquid chromatography (HPLC) and sodium dodecyl sulfate-polyacrylamide gel electrophoresis (SDS-PAGE) followed by phosphor imager analysis for integrity, and by a cell-binding assay for immunoreactivity. ITLC analyses of radiolabelled DN30 was performed on silica gel impregnated glass fibre sheets (Pall Corp., East Hills, NY). As the mobile phase, 0.02 M citrate buffer (pH 5.0) was used.

HPLC monitoring of the final products was performed on a Jasco HPLC system using a Superdex^{™} 200 10/300 GL size exclusion column (GE Healthcare Life Sciences). As eluent a mixture of 0.05 M sodium phosphate and 0.15 M sodium chloride (pH 6.8) was used at a flow rate of 0.5 mL/min. Electrophoresis was performed on a Phastgel System (GE Healthcare Life Sciences) using preformed 7.5% SDS-PAGE gels under nonreducing conditions.

The immunoreactivity was determined by measuring binding of ⁸⁹Zr-DN30 and ¹³¹I-DN30 (10000 cpm/mL) to a serial dilution of 2% paraformaldehyde-fixed GTL-16 cells essentially as described by Lindmo et al., J Immunol Methods 1984; 72:77-89. Data were graphically analysed in a modified Lineweaver-Burk (double-reciprocal) plot and the immunoreactivity was determined by extrapolating to conditions representing infinite antigen excess.

### Biodistribution

Nude mice bearing subcutaneously implanted xenografts of the human gastric carcinoma cell line GTL-16 or the HNSCC cell line FaDu were used. Female mice (HSD:Athymic Nude-*Foxn1^{nu}*, 21-31 g, Harlan CPB) were 10-14 weeks old at the time of the experiment. All animal experiments were performed according to National Institutes of Health principles of laboratory animal care and Dutch national law ("Wet op de dierproeven", Stb 1985, 336).

Three sets of biodistribution studies were performed. In the first experiment, the biodistribution of intravenous (i.v.) coinjected ⁸⁹Zr-DN30 (0.28 ± 0.004 MBq) and ¹³¹I-DN30 (0.37 ± 0.004 MBq) was assessed in GTL-16 bearing nude mice. Unlabeled DN30 was added to the injection mixture so that each of the animals received 100 µg of MAb in total. This initial antibody dose was chosen sufficiently high to prevent rapid IgG₂ₐ isotype-related elimination of the MAb from the blood as has been described for the nude mouse model, and sufficiently low to prevent antigen saturation in the tumour. The mean tumor size at the start of the experiment was 64 ± 33 mm³. At 1, 2, 3 and 5 days post injection (p.i.), 4 mice per time point were anaesthetised, bled, killed, and dissected. After blood, tumour, and normal tissues had been weighed, the amount of radioactivity in each sample was measured in a gamma counter. Radioactivity uptake was calculated as the percentage of the injected dose per gram of tissue (%ID/g) .

In the second experiment, the relation between protein dose of ⁸⁹Zr-DN30 and biodistribution was investigated in GTL-16 bearing nude mice. Four groups of 4 mice received 0.39 ± 0.02 MBq ⁸⁹Zr-DN30 by i.v. injection. Unlabelled DN30 was added to the injection mixture so that per group the animals received 25, 50, 100, or 200 µg DN30 in total, respectively. The mean tumor size at the start of the experiment was 50 ± 34 mm³, and was similar for the different groups. At day 3 p.i., all animals were anaesthetised, bled, killed, and dissected, with further processing according to the above procedure.

In the third experiment, biodistribution of ⁸⁹Zr-DN30 (0.28 ± 0.01 MBq; 100 µg) was assessed in FaDu bearing nude mice (n = 16). The mean tumor size at the start of the experiment was 149 ± 47 mm³. At 1, 2, 3, and 4 days p.i., the mice were anaesthetised, bled, killed, and dissected, with further processing according to the above procedure.

### PET imaging procedures

Animal PET studies for imaging and quantification of tumor targeting of ⁸⁹Zr-DN30 were performed essentially as described by Verel et al., J Nucl Med 2003; 44:1663-70. Briefly, PET studies were performed using a double-crystal-layer HRRT PET scanner (Siemens/CTI Knoxville), a dedicated small animal and human brain scanner (De Jong HWAM et al., Phys Med Biol 2007; 52:1505-26). Four GTL-16 xenograft-bearing nude mice were injected i.v. with 2.6 ± 0.04 MBq ⁸⁹Zr- DN3 (100 µg). The mean tumor size at the start of the experiment was 46 ± 19 mm³. The animals were sedated using isoflurane and imaged at 10 min, and at 1, 2, 3, and 4 days p.i. 3D emission scans were acquired in 64-bit list mode during 60 min using a 400-650 keV window. The 64-bit list mode file was first converted into a single-frame histogram using a span of 9, and subsequently reconstructed using a 3D OP-OSEM reconstruction with 2 iterations and 16 subsets. After reconstruction, regions of interest (ROI) were drawn semiautomatic around the tumours using a 3D isocontour at 50% of maximum pixel value with background correction. Immediately after the last PET scan the animals were killed, dissected, and processed as described above.

In a second imaging study, a group of four FaDu xenograft-bearing nude mice were injected i.v. with 1.8 ± 0.01 MBq ⁸⁹Zr-DN30 (100 µg). The mean tumor size at the start of the experiment was 231 ± 64 mm³. Imaging was performed as described above.

### Statistical analyses

Differences in tissue uptake between coinjected conjugates were statistically analyzed for each time point with SPSS 11.0 software using Student *t*-test for paired data. Two-sided significance levels were calculated and *P* <0.01 was considered statistically significant. Statistical analysis of differences in tissue uptake between different groups was performed using Student *t*-test for unpaired data. Regression analysis of PET-defined ⁸⁹Zr tumour uptake versus ex vivo assessed ⁸⁹Zr tumour uptake was also performed with SPSS 11.0 software.

### RESULTS

### Immunohistochemistry

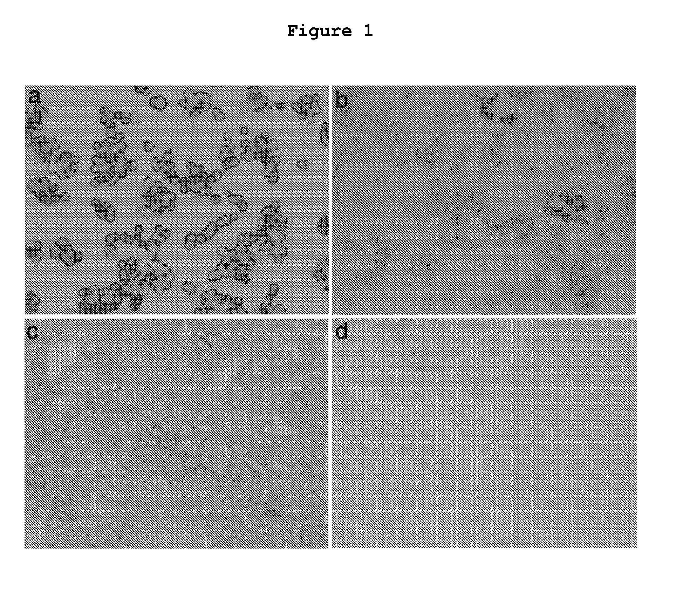

The cell lines GTL-16 and FaDu both expressed Met (Fig. 1a and 1b), but Met expression was highest in GTL-16 (∼100000 copies at outer cell surface). Met copy number could not accurately be determined for FaDu. Higher Met expression was also found for GLT-16 xenografts in comparison with FaDu xenografts (Fig. 1c and 1d).

### Radiolabelling

Radiolabelling of DN30 with ¹³¹I or *N*-sucDf-DN30 with ⁸⁹Zr resulted in overall labelling yields of >85% and >70%, respectively. The radiochemical purity, as determined by ITLC, was always higher than 95% for both products. The specific radioactivities of the final products ranged from 30 to 80 MBq/mg for ¹³¹I-DN30 and from 54 to 70 MBq/mg for ⁸⁹Zr-DN30. HPLC and SDS-PAGE analysis revealed optimal integrity of DN30, irrespective of whether the MAb was labelled with ¹³¹I or ⁸⁹Zr. The immunoreactivity of both radioimmunoconjugates was always >70% at the highest cell concentration and >95% at infinite antigen excess.

### Biodistribution

In the first study, the inventors compared the biodistribution of co-injected ⁸⁹Zr-DN30 and ¹³¹I-DN30 (100 µg DN30 total) in GTL-16-tumour-bearing nude mice up to 5 days after injection. ⁸⁹Zr-DN30 showed a significantly higher tumour accumulation, as well as a significantly higher liver and spleen uptake compared to the ¹³¹I-labeled counterpart (Table 1). The ⁸⁹Zr-DN30 tumour accumulation ranged from 12.2 ± 4.3 %ID/g to 19.6 ± 3.3 %ID/g, and the ¹³¹I-DN30 accumulation from 7.8 ± 3.1 %ID/g to 5.3 ± 1.0 %ID/g, in the time period between 1 and 5 days p.i.. Small differences in uptake of both conjugates were found for blood and all other normal tissues. Nevertheless, tumour-to-normal tissue ratios were always higher for ⁸⁹Zr-DN30, except for liver and spleen at the earliest time points. In contrast to ⁸⁹Zr, levels of ¹³¹I in tumours never exceeded levels of ¹³¹I in blood. On the basis of these results, the inventors selected ⁸⁹Zr-DN30 for the remaining biodistribution and PET imaging studies.

**Table 1**

| **Biodistribution time** | **1 d** | **2 d** | **3 d** | **5 d** |
|---|---|---|---|---|
| *Uptake of ⁸⁹Zr-DN30 [%ID*/*g]^{a}* | | | | |
| Blood | 17.1 (2.1) | 13.3 (1.0)* | 10.6 (3.2)* | 8.8 (2.3)* |
| Tumour (GTL-16) | 12.2 (4.3)* | 17.3 (4.5)* | 18.1 (4.5)* | 19.6 (3.3)* |
| Sternum | 2.89 (0.3) | 3.1 (0.5)* | 3.1 (0.3)* | 2.2 (0.2)* |
| Heart | 4.2 (0.6) | 3.5 (0.6) | 2.8 (0.6) | 3.7 (1.0) |
| Lung | 6.2 (0.4) | 5.7 (0.2) | 4.5 (0.7) | 3.7 (0.6) |
| Liver | 6.1 (0.3)* | 6.3 (0.2)* | 8.4 (2.4)* | 7.2 (0.7)* |
| Spleen | 6.6 (1.1)* | 8.6 (0.9)* | 7.9 (1.6)* | 7.4 (1.8)* |
| Kidney | 4.0 (0.6) | 3.9 (0.2)* | 3.5 (0.2) | 3.1 (0.2)* |
| Bladder | 3.3 (0.6) | 3.3 (0.4) | 3.2 (0.3) | 2.9 (0.3) |
| Muscle | 1.2 (0.2) | 1.2 (0.1) | 1.2 (0.2) | 0.6 (0.2) |
| Colon | 1.5 (0.2)* | 1.9 (0.2) | 1.8 (0.7) | 1.3 (0.4) |
| Ileum | 1.8 (0.3) | 2.5 (0.7) | 2.4 (1.2) | 2.2 (0.8) |
| Stomach | 1.5 (0.1) | 1.7 (0.2) | 1.5 (0.3) | 1.6 (0.5) |

| | | | | |
|---|---|---|---|---|
| *Uptake of ¹³¹I-DN30 [%ID*/*g]^{a}* | | | | |
| Blood | 17.6 (2.1) | 15.0 (0.9) | 12.4 (3.2) | 9.9 (2.0) |
| Tumour (GTL-16) | 7.8 (3.1) | 7.8 (1.1) | 6.7 (1.5) | 5.3 (1.0) |
| Sternum | 2.8 (0.3) | 2.3 (0.5) | 2.1 (0.4) | 1.2 (0.3) |
| Heart | 4.5 (0.7) | 3.9 (0.7) | 3.0 (0.8) | 3.9 (0.9) |
| Lung | 6.7 (0.3) | 6.3 (0.3) | 4.8 (0.7) | 3.5 (0.6) |
| Liver | 2.9 (0.5) | 2.8 (0.3) | 3.1 (0.8) | 1.8 (0.1) |
| Spleen | 3.8 (0.3) | 4.2 (0.6) | 4.0 (0.9) | 2.2 (0.2) |
| Kidney | 4.0 (0.8) | 3.8 (0.2) | 3.0 (0.4) | 2.1 (0.3) |
| Bladder | 4.2 (0.7) | 4.7 (1.1) | 3.5 (0.5) | 2.7 (0.3) |
| Muscle | 1.3 (0.2) | 1.4 (0.3) | 1.2 (0.2) | 0.6 (0.1) |
| Colon | 1.6 (0.2) | 1.8 (0.1) | 1.6 (0.4) | 0.9 (0.2) |
| Ileum | 1.8 (0.3) | 2.1 (0.4) | 1.7 (0.5) | 1.2 (0.2) |
| Stomach | 2.6 (0.7) | 2.1 (0.2) | 1.9 (0.3) | 1.2 (0.2) |

| | | | | |
|---|---|---|---|---|
| * Significant differences (P<0.01) between ⁸⁹Zr-DN30 and ¹³¹I-DN30 uptake are marked with an asterisk. ^{a}All data are presented as mean ± S.D. (n=4). | | | | |

To investigate if 100 µg is an appropriate protein dose of MAb DN30 for efficient tumour targeting in mice, the biodistribution of ⁸⁹Zr-DN30 was assessed for four protein doses ranging from 25 - 200 µg in GTL-16 xenograft-bearing nude mice at day 3 p.i.. The average uptake levels in blood, tumour, and normal tissues, and tumour-to-normal tissue ratios are shown in Table 2. Tumour uptake levels combined with tumour-to-normal tissue ratios were considered most favourable for the 50 and 100 µg group. At the lowest protein dose, blood levels of DN30 were relatively low and strongly variable between mice, while e.g. spleen uptake was high, which is indicative for rapid IgG₂ₐ isotype-related elimination of MAbs in the nude mouse model. Therefore, the inventors used the 100 µg MAb dose in the subsequent biodistribution and imaging studies.

**Table 2**

| **Protein dose** | **25 µg** | **50 µg** | **100 µg** | **200 µg** |
|---|---|---|---|---|
| *Uptake of ⁸⁹Zr-DN30 [%ID*/*g]^{a}* | | | | |
| Blood* | 2.8 (4.4) | 5.6 (1.8) | 8.9 (2.7) | 10.2 (1.0) |
| Tumour | 14.3 (3.9) | 16.6 (2.7) | 18.4 (5.0) | 15.1 (3.7) |
| Sternum | 2.9 (0.2) | 2.4 (0.3) | 2.7 (0.4) | 2.5 (0.3) |
| Heart | 1.6 (1.1) | 1.8 (0.3) | 2.4 (0.4) | 2.7 (0.5) |
| Lung | 2.0 (1.4) | 3.1 (0.7) | 4.4 (1.0) | 4.4 (0.3) |
| Liver | 9.9 (2.4) | 7.4 (1.1) | 8.0 (2.0) | 9.0 (5.1) |
| Spleen | 11.0 (5.3) | 8.4 (2.6) | 8.0 (3.4) | 6.6 (0.3) |
| Kidney | 2.2 (0.2) | 2.6 (0.2) | 3.2 (0.1) | 3.3 (0.3) |
| Muscle | 0.7 (0.2) | 0.8 (0.1) | 1.1 (0.1) | 1.0 (0.1) |
| | | | | |
| *Tumour*/*Tissue ratios* | | | | |
| T/Blood | 5.0 | 2.9 | 2.1 | 1.5 |
| T/Sternum | 5.0 | 6.8 | 6.9 | 6.0 |
| T/Heart | 9.1 | 9.4 | 7.5 | 5.5 |
| T/Lung | 7.3 | 5.4 | 4.2 | 3.4 |
| T/Liver | 1.4 | 2.2 | 2.3 | 1.7 |
| T/Spleen | 1.3 | 2.0 | 2.3 | 2.3 |
| T/Kidney | 6.5 | 6.5 | 5.7 | 4.5 |
| T/Muscle | 21.5 | 20.1 | 17.5 | 15.4 |

| | | | | |
|---|---|---|---|---|
| ***** Significant differences (P<0.01) in ⁸⁹Zr-DN30 uptake between the given protein doses are marked with an asterisk. ^{a}All data are presented as mean ± S.D. (n=4). | | | | |

In the third experiment, biodistribution of ⁸⁹Zr-DN30 was assessed in FaDu (low Met expression)-bearing nude mice up to 4 days p.i. (Table 3). Uptake of ⁸⁹Zr-DN30 in FaDu tumours was significantly lower than that in GTL-16 tumours, e.g. at 3 days p.i. the FaDu tumour uptake was 7.8 ± 1.2 %ID/g compared to 18.1 ± 4.5 %ID/g in the GTL-16 tumours (P <0.01). This lower uptake correlates with the different expression levels of Met in these tumours as determined by immunohistochemistry (Fig. 1).

**Table 3**

| **Biodistribution time** | **1 d** | **2 d** | **3 d** | **4 d** |
|---|---|---|---|---|
| *Uptake of ⁸⁹Zr-DN30 [%ID*/*g]^{a}* | | | | |
| Blood | 14.4 (1.0) | 11.9 (1.5) | 10.3 (1.5) | 9.3 (2.3) |
| Tumour (FaDu) | 6.3 (0.9) | 9.3 (1.0) | 7.8 (1.2) | 6.7 (0.3) |
| Sternum | 2.5 (0.3) | 2.7 (0.1) | 2.5 (0.2) | 3.0 (0.4) |
| Heart | 3.3 (0.5) | 3.3 (0.4) | 2.9 (0.4) | 2.7 (1.0) |
| Lung | 5.7 (0.8) | 5.3 (0.4) | 4.5 (0.2) | 4.8 (2.0) |
| Liver | 4.1 (0.9) | 4.8 (0.8) | 4.7 (0.7) | 4.9 (0.1) |
| Spleen | 4.5 (0.7) | 5.4 (1.4) | 4.3 (0.8) | 5.9 (0.9) |
| Kidney | 3.3 (0.6) | 3.6 (0.3) | 3.1 (0.4) | 3.0 (0.3) |
| Bladder | 3.3 (0.8) | 3.4 (0.3) | 3.1 (0.3) | 2.6 (0.6) |
| Muscle | 1.3 (0.2) | 1.3 (0.01) | 1.1 (0.2) | 0.9 (0.1) |
| Colon | 1.6 (0.2) | 2.0 (0.2) | 1.3 (0.4) | 1.0 (0.01) |
| Ileum | 1.9 (0.5) | 2.6 (0.7) | 1.5 (0.5) | 1.2 (0.1) |
| Stomach | 1.6 (0.4) | 1.6 (0.1) | 1.3 (0.2) | 1.0 (0.2) |

| | | | | |
|---|---|---|---|---|
| ^{a} All data are presented as mean ± S.D. (n=4). | | | | |

### PET imaging

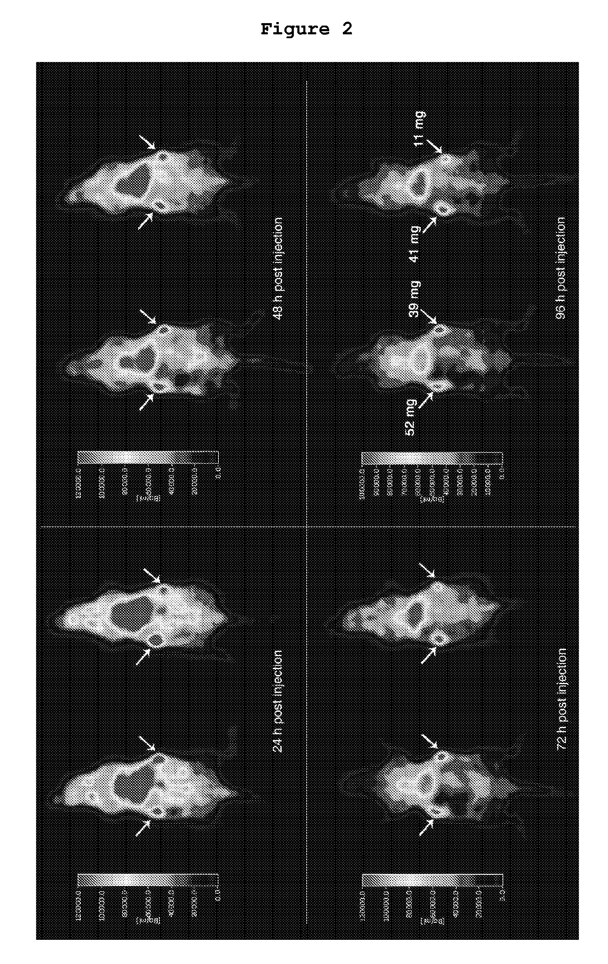

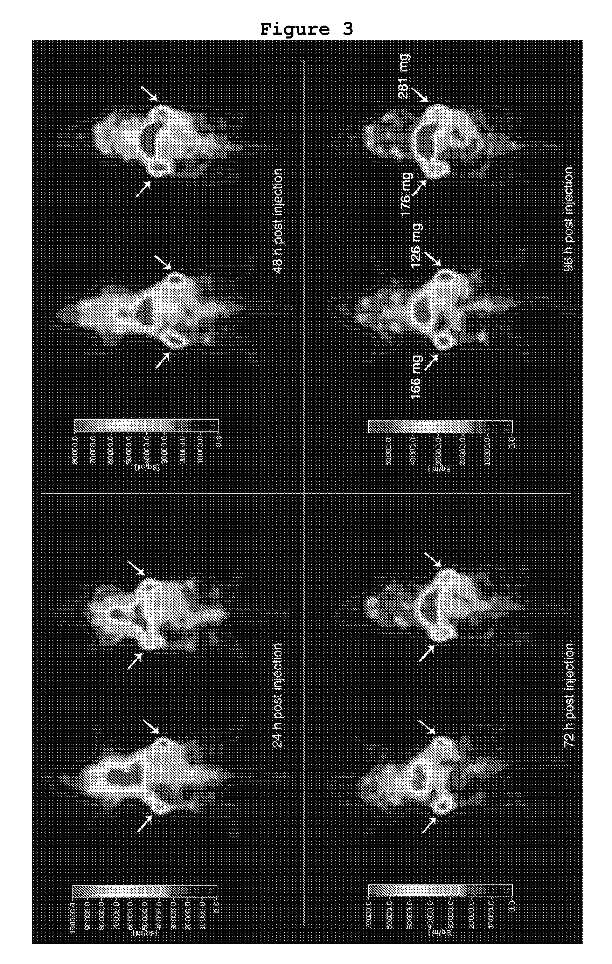

Representative PET images of mice bearing Met expressing human cancer xenografts, between 1 and 4 days after i.v. injection of ⁸⁹Zr-DN30, are shown in Fig. 2 (GTL-16) and Fig. 3 (FaDu). At the earliest imaging time point, 10 min p.i., only activity in the blood pool was observed (scans not shown). All tumours (arrows) could be clearly visualized in both xenograft hosts as early as 1 day p.i., and good delineation of the tumours persisted through the final imaging session. As expected from the biodistribution data, tumour uptake was more pronounced in the GTL-16 xenografts. Tumour localization was obvious along with some blood pool, which diminished over time, and liver and spleen uptake. Noteworthy, GTL-16 tumours as small as 11 mg were clearly visualized. A good correlation was found between PET-defined tumour uptake data and ex vivo tumour uptake measurements (R²=0.98).

Naturally, while the principle of the invention remains the same, the details of construction and the embodiments may widely vary with respect to what has been described and illustrated purely by way of example, without departing from the scope of the present invention as defined in the appended claims.

## Claims

1. An immuno-imaging agent for the detection of tumor cells by means of an immuno-imaging technique including at least one of:
- an anti-Met monoclonal antibody,
- a fragment of an anti-Met monoclonal antibody containing the epitope binding region thereof,
- a genetically engineered antibody containing the epitope binding region or Complementary Determining Regions of an anti-Met monoclonal antibody,
- a humanized antibody containing the epitope binding region or Complementary Determining Regions of an anti-Met monoclonal antibody, or combinations thereof,
**characterized in that** said anti-Met monoclonal antibody is produced by the hybridoma cell line ICLC PD 05006.

2. The immuno-imaging agent according to claim 1, wherein said immuno-imaging technique is selected among gamma camera imaging technique, PET-technique and MRI-technique.

3. The immuno-imaging agent according to claim 1, wherein said immuno-imaging agent is coupled directly or indirectly to a detectable signaling moiety, wherein said detectable signaling moiety is active or activatable.

4. The immuno-imaging agent according to claim 1, wherein said immuno-imaging agent is coupled to a molecule suitable to be subsequently coupled to a detectable signaling moiety, wherein said detectable signaling moiety is active or activatable.

5. The immuno-imaging agent according to claim 3 or claim 4, wherein said detectable signaling moiety is selected among a gamma camera-imageable agent, a PET-imageable agent, a MRI-imageable agent.

6. The immuno-imaging agent according to claim 5, wherein said gamma camera-imageable agent is selected from ³H, ¹⁴C, ³⁵S, ^{99m}Tc, ¹²³I, ¹²⁵I, ¹³¹I, ¹¹¹In, ⁹⁷Ru, ⁶⁷Ga, and ²⁰¹Tl, ¹⁸⁶Re, ¹⁷⁷Lu.

7. The immuno-imaging agent according to claim 5, wherein said PET-imageable agent is selected from ⁸⁹Zr, ¹²⁴I ⁶⁴Cu, ⁷⁶Br, ⁸⁶Y, ¹⁸F, ⁶⁸Ga, ⁴⁵Ti.

8. The immuno-imaging agent according to claim 5, said MRI-imageable agent is selected from Ga, Mn, Cu, Fe, Au, and Eu.

9. The immuno-imaging agent according to any one of claims 1 to 8, wherein said anti-Met monoclonal antibody presents a nucleotide sequence comprising SEQ ID NO.:1 and SEQ ID NO.:2, or a nucleotide sequence comprising SEQ ID NO.:1 and SEQ ID NO.:2 wherein at least one conservative substitution is present.

10. The immuno-imaging agent according to any one of claims 1 to 8, wherein said fragment containing the epitope binding region of said anti-Met monoclonal antibody is selected from Fab, F(ab')₂, Fab', Fv, scFv.

11. The immuno-imaging agent according to any one of claims 1 to 8, wherein said genetically engineered antibody containing the epitope binding region or Complementary Determining Regions of said anti-Met monoclonal antibody presents a nucleotide sequence comprising SEQ ID NO.:8 (CDR-H1), SEQ ID NO.:9 (CDR-H2), SEQ ID NO.:10 (CDR-H3), SEQ ID NO.:11 (CDR-L1), SEQ ID NO.:12 (CDR-L2) and SEQ ID NO.:13 (CDR-L3).

12. The immuno-imaging agent according to any one of claims 1 to 8, wherein said humanized antibody containing the epitope binding region or Complementary Determining Regions of said anti-Met monoclonal antibody is a mouse/human chimeric antibody.

13. A diagnostic composition suitable for use in an immuno-imaging technique comprising:
(i) an immuno-imaging agent including at least one of:
- an anti-Met monoclonal antibody,
- a fragment of an anti-Met monoclonal antibody containing the epitope binding region thereof,
- a genetically engineered antibody containing the epitope binding region or Complementary Determining Regions of an anti-Met monoclonal antibody,
- a humanized antibody containing the epitope binding region or Complementary Determining Regions of an anti-Met monoclonal antibody, or combinations thereof,
wherein said immuno-imaging agent a) is coupled directly or indirectly to a detectable signaling moiety, or b) is coupled to a molecule suitable to be subsequently coupled to a detectable signaling moiety, wherein said detectable signaling moiety is active or activatable, and
(ii) a diagnostically acceptable carrier and/or excipient,
**characterized in that** said anti-Met monoclonal antibody is produced by the hybridoma cell line ICLC PD 05006.

14. Composition according to claim 13, wherein said immuno-imaging technique is selected from gamma camera imaging technique, PET-technique and MRI-technique.

15. Composition according to claim 13, wherein said detectable signaling moiety is selected from a gamma camera-imageable agent, a PET-imageable agent, a MRI-imageable agent.

16. Composition according to claim 15, wherein said gamma camera-imageable agent is selected from ³H, ¹⁴C, ³⁵S, ^{99m}Tc, ¹²³I, ¹²⁵I, ¹³¹I, ¹¹¹In, ⁹⁷Ru_{,} ⁶⁷Ga, ²⁰¹Tl, ¹⁸⁶Re, and ¹⁷⁷Lu.

17. Composition according to claim 15, wherein said PET-imageable agent is selected from ⁸⁹Zr, ¹²⁴I, ⁶⁴Cu, ⁷⁶Br, ⁸⁶Y, ¹⁸F, ⁶⁸Ga, and ⁴⁵Ti.

18. Composition according to claim 15, said MRI-imageable agent is selected from Ga, Mn, Cu, Fe, Au, and Eu.

19. Composition according to any one of claims 13 to 18, wherein said anti-Met monoclonal antibody presents a nucleotide sequence comprising SEQ ID NO.:1 and SEQ ID NO.:2, or a nucleotide sequence comprising SEQ ID NO.:1 and SEQ ID NO.:2 wherein at least one conservative substitution is present.

20. Composition according to any one of claims 13 to 18, wherein said fragment containing the epitope binding region of said anti-Met monoclonal antibody is selected from Fab, F(ab')₂, Fab' , Fv, scFv.

21. Composition according to any one of claims 13 to 18, wherein said genetically engineered antibody containing the epitope binding region or Complementary Determining Regions of said anti-Met monoclonal antibody presents a nucleotide sequence comprising SEQ ID NO.:8 (CDR-H1), SEQ ID NO.:9 (CDR-H2), SEQ ID NO.:10 (CDR-H3), SEQ ID NO.:11 (CDR-L1), SEQ ID NO.:12 (CDR-L2) and SEQ ID NO.:13 (CDR-L3).

22. Composition according to any one of claims 12 13 to 18, wherein said humanized antibody containing the epitope binding region or Complementary Determining Regions of said anti-Met monoclonal antibody is a mouse/human chimeric antibody.

23. Diagnostic kit comprising a first vial containing an immuno-imaging agent according to any one of claims 1 to 12 or a diagnostic composition according to any one of claims 13 to 22, and instructions for using said immuno-imaging agent or said diagnostic composition for the diagnosis of a cancerous condition or a tumor in a subject, wherein said cancer or tumor cells express Met.

24. Diagnostic kit according to claim 23, wherein said first vial contains said immuno-imaging agent coupled to a molecule suitable to be coupled to a detectable signaling moiety, and said kit comprises a second vial comprising said detectable signaling moiety suitable to be coupled to said immuno-imaging agent.


SEQUENCE LISTING

<110>  Metheresis Translational Research SA

<120>  Anti-Met monoclonal antibody, fragments and derivatives thereof
       for use in tumor diagnosis, corresponding compositions and kits

<130>  BEP9657-CF

<160>  13
<170>  PatentIn version 3.3

<210>  1
<211>  1386
<212>  DNA
<213>  artificial

<220>
<223>  AntiMET-R heavy chain

<400>  1
atgggatgga gctatatcat cctctttttg gtagcaacag ctacagatgg ccactcccag      60

gtccaactgc agcagcctgg gactgaactg gtgaagcctg gggcttcagt gaagctgtcc     120

tgcaaggctt ctggctacac cttcaccagt tactggatac actgggtgaa gcagaggcct     180

ggacaaggcc ttgagtggat tggagagatt aatcctagca gcggtcgtac taactacaac     240

gagaaattca agaacaaggt cacagtgact gtagacaaat cttccaccac agcctacatg     300

caactcagca acctgacatc tgaggactct gcggtctatt actgtgcaag taggggctac     360

tggggccaag gcaccactct cacagtctcc tcagccaaaa caacagcccc atcggtctat     420

ccactggccc ctgtgtgtgg aaatacaact ggctcctcgg tgactctagg atgcctggtc     480

aagggttatt tccctgagcc agtgaccttg acctggaact ctggatccct gtccagtggt     540

gtgcacacct tcccagctgt cctgcagtct gacctctaca ccctcagcag ctcagtgact     600

gtaacctcga gcacctggcc cagccagtcc atcacctgca atgtggccca cccggcaagc     660

agcaccaagg tggacaagaa aattgagccc agagggccca caatcaagcc ctgtcctcca     720

tgcaaatgcc cagcacctaa cctcttgggt ggaccatccg tcttcatctt ccctccaaag     780

atcaaggatg tactcatgat ctccctgagc cccatagtca catgtgtggt ggtggatgtg     840

agcgaggatg acccagatgt ccagatcagc tggtttgtga acaacgtgga agtacacaca     900

gctcagacac aaacccatag agaggattac aacagtactc tccgggtggt cagtgccctc     960

cccatccagc accaggactg gatgagtggc aaggagttca aatgcaaggt caacaacaaa    1020

gacctcccag cgcccatcga gagaaccatc tcaaaaccca aagggtcagt aagagctcca    1080

caggtatatg tcttgcctcc accagaagaa gagatgacta agaaacaggt cactctgacc    1140

tgcatggtca cagacttcat gcctgaagac atttacgtgg agtggaccaa caacgggaaa    1200

acagagctaa actacaagaa cactgaacca gtcctggact ctgatggttc ttacttcatg    1260

tacagcaagc tgagagtgga aaagaagaac tgggtggaaa gaaatagcta ctcctgttca    1320

gtggtccacg agggtctgca caatcaccac acgactaaga gcttctcccg gactccgggt    1380

aaatga                                                               1386

<210>  2
<211>  717
<212>  DNA
<213>  artificial

<220>
<223>  AntiMET-R light chain

<400>  2
atggagacag acacaatcct gctatgggtg ctgctgctct gggttccagg ctccactggt      60

gacattgtgc tgacccaatc tccagcttct ttggctgtgt ctctagggca gagggccacc     120

atctcctgca aggccagcca aagtgttgat tatgatggtg gtagttatat gagttggttc     180

caacagagac caggacagcc acccaaactc ctcatctctg ctgcatccaa tctagaatct     240

gggatcccag ccaggtttag tggcagtggc tctgggacag acttcaccct caatatccat     300

cctgtggagg aggaggatgt tgcaacctat tactgtcagc aaagttatga ggatccgctc     360

acgttcggtg ctgggaccaa gctggagctg aaacgggctg atgctgcacc aactgtatcc     420

atcttcccac catccagtga gcagttaaca tctggaggtg cctcagtcgt gtgcttcttg     480

aacaacttct accccaaaga catcaatgtc aagtggaaga ttgatggcag tgaacgacaa     540

aatggcgtcc tgaacagttg gactgatcag gacagcaaag acagcaccta cagcatgagc     600

agcaccctca cgttgaccaa ggacgagtat gaacgacata acagctatac ctgtgaggcc     660

actcacaaga catctacttc acccattgtc aagagcttca acaggaatga gtgttag        717


<210>  3
<211>  1473
<212>  DNA
<213>  artificial

<220>
<223>  Anti-MET-R Heavy chain with a tag sequence to the 3'

<400>  3
atgggatgga gctatatcat cctctttttg gtagcaacag ctacagatgg ccactcccag      60

gtccaactgc agcagcctgg gactgaactg gtgaagcctg gggcttcagt gaagctgtcc     120

tgcaaggctt ctggctacac cttcaccagt tactggatac actgggtgaa gcagaggcct     180

ggacaaggcc ttgagtggat tggagagatt aatcctagca gcggtcgtac taactacaac     240

gagaaattca agaacaaggt cacagtgact gtagacaaat cttccaccac agcctacatg     300

caactcagca acctgacatc tgaggactct gcggtctatt actgtgcaag taggggctac     360

tggggccaag gcaccactct cacagtctcc tcagccaaaa caacagcccc atcggtctat     420

ccactggccc ctgtgtgtgg aaatacaact ggctcctcgg tgactctagg atgcctggtc     480

aagggttatt tccctgagcc agtgaccttg acctggaact ctggatccct gtccagtggt     540

gtgcacacct tcccagctgt cctgcagtct gacctctaca ccctcagcag ctcagtgact     600

gtaacctcga gcacctggcc cagccagtcc atcacctgca atgtggccca cccggcaagc     660

agcaccaagg tggacaagaa aattgagccc agagggccca caatcaagcc ctgtcctcca     720

tgcaaatgcc cagcacctaa cctcttgggt ggaccatccg tcttcatctt ccctccaaag     780

atcaaggatg tactcatgat ctccctgagc cccatagtca catgtgtggt ggtggatgtg     840

agcgaggatg acccagatgt ccagatcagc tggtttgtga acaacgtgga agtacacaca 900

gctcagacac aaacccatag agaggattac aacagtactc tccgggtggt cagtgccctc 960

cccatccagc accaggactg gatgagtggc aaggagttca aatgcaaggt caacaacaaa 1020

gacctcccag cgcccatcga gagaaccatc tcaaaaccca aagggtcagt aagagctcca 1080

caggtatatg tcttgcctcc accagaagaa gagatgacta agaaacaggt cactctgacc 1140

tgcatggtca cagacttcat gcctgaagac atttacgtgg agtggaccaa caacgggaaa 1200

acagagctaa actacaagaa cactgaacca gtcctggact ctgatggttc ttacttcatg 1260

tacagcaagc tgagagtgga aaagaagaac tgggtggaaa gaaatagcta ctcctgttca 1320

gtggtccacg agggtctgca caatcaccac acgactaaga gcttctcccg gactccgggt 1380

aaagctagct ctgactacaa ggacgacgat gacaagagcg attacaaaga cgatgatgat 1440

aagctgcagc atcaccacca tcatcaccat tga 1473

<210> 4
<211> 9
<212> DNA
<213> artificial

<220>
<223> synthetic promoter - upstream

<400> 4
acctgggtt 9

<210> 5
<211> 13
<212> DNA
<213> artificial

<220>
<223> synthetic promoter - downstream

<400> 5
attggttggt tgg 13

<210> 6
<211> 461
<212> PRT
<213> artificial

<220>
<223> AntiMET-R heavy chain

<400> 6

Met Gly Trp Ser Tyr Ile Ile Leu Phe Leu Val Ala Thr Ala Thr Asp
1               5                   10                  15

Gly His Ser Gln Val Gln Leu Gln Gln Pro Gly Thr Glu Leu Val Lys
            20                  25                  30

Pro Gly Ala Ser Val Lys Leu Ser Cys Lys Ala Ser Gly Tyr Thr Phe
        35                  40                  45

Thr Ser Tyr Trp Ile His Trp Val Lys Gln Arg Pro Gly Gln Gly Leu
    50                  55                  60

Glu Trp Ile Gly Glu Ile Asn Pro Ser Ser Gly Arg Thr Asn Tyr Asn
65                  70                  75                  80

Glu Lys Phe Lys Asn Lys Val Thr Val Thr Val Asp Lys Ser Ser Thr
                85                  90                  95

Thr Ala Tyr Met Gln Leu Ser Asn Leu Thr Ser Glu Asp Ser Ala Val
            100                 105                 110

Tyr Tyr Cys Ala Ser Arg Gly Tyr Trp Gly Gln Gly Thr Thr Leu Thr
        115                 120                 125

Val Ser Ser Ala Lys Thr Thr Ala Pro Ser Val Tyr Pro Leu Ala Pro
    130                 135                 140

Val Cys Gly Asn Thr Thr Gly Ser Ser Val Thr Leu Gly Cys Leu Val
145                 150                 155                 160

Lys Gly Tyr Phe Pro Glu Pro Val Thr Leu Thr Trp Asn Ser Gly Ser
                165                 170                 175

Leu Ser Ser Gly Val His Thr Phe Pro Ala Val Leu Gln Ser Asp Leu
            180                 185                 190

Tyr Thr Leu Ser Ser Ser Val Thr Val Thr Ser Ser Thr Trp Pro Ser
        195                 200                 205

Gln Ser Ile Thr Cys Asn Val Ala His Pro Ala Ser Ser Thr Lys Val
    210                 215                 220

Asp Lys Lys Ile Glu Pro Arg Gly Pro Thr Ile Lys Pro Cys Pro Pro
225                 230                 235                 240

Cys Lys Cys Pro Ala Pro Asn Leu Leu Gly Gly Pro Ser Val Phe Ile
                245                 250                 255

Phe Pro Pro Lys Ile Lys Asp Val Leu Met Ile Ser Leu Ser Pro Ile
            260                 265                 270

Val Thr Cys Val Val Val Asp Val Ser Glu Asp Asp Pro Asp Val Gln
        275                 280                 285

Ile Ser Trp Phe Val Asn Asn Val Glu Val His Thr Ala Gln Thr Gln
    290                 295                 300

Thr His Arg Glu Asp Tyr Asn Ser Thr Leu Arg Val Val Ser Ala Leu
305                 310                 315                 320

Pro Ile Gln His Gln Asp Trp Met Ser Gly Lys Glu Phe Lys Cys Lys
                325                 330                 335

Val Asn Asn Lys Asp Leu Pro Ala Pro Ile Glu Arg Thr Ile Ser Lys
            340                 345                 350

Pro Lys Gly Ser Val Arg Ala Pro Gln Val Tyr Val Leu Pro Pro Pro
        355                 360                 365

Glu Glu Glu Met Thr Lys Lys Gln Val Thr Leu Thr Cys Met Val Thr
    370                 375                 380

Asp Phe Met Pro Glu Asp Ile Tyr Val Glu Trp Thr Asn Asn Gly Lys
385                 390                 395                 400

Thr Glu Leu Asn Tyr Lys Asn Thr Glu Pro Val Leu Asp Ser Asp Gly
                405                 410                 415

Ser Tyr Phe Met Tyr Ser Lys Leu Arg Val Glu Lys Lys Asn Trp Val
            420                 425                 430

Glu Arg Asn Ser Tyr Ser Cys Ser Val Val His Glu Gly Leu His Asn
        435                 440                 445

His His Thr Thr Lys Ser Phe Ser Arg Thr Pro Gly Lys

450                 455                 460

<210>  7
<211>  238
<212>  PRT
<213>  artificial

<220>
<223>  AntiMET-R light chain

<400>  7

Met Glu Thr Asp Thr Ile Leu Leu Trp Val Leu Leu Leu Trp Val Pro
1               5                   10                  15

Gly Ser Thr Gly Asp Ile Val Leu Thr Gln Ser Pro Ala Ser Leu Ala
            20                  25                  30

Val Ser Leu Gly Gln Arg Ala Thr Ile Ser Cys Lys Ala Ser Gln Ser
        35                  40                  45

Val Asp Tyr Asp Gly Gly Ser Tyr Met Ser Trp Phe Gln Gln Arg Pro
    50                  55                  60

Gly Gln Pro Pro Lys Leu Leu Ile Ser Ala Ala Ser Asn Leu Glu Ser
65                  70                  75                  80

Gly Ile Pro Ala Arg Phe Ser Gly Ser Gly Ser Gly Thr Asp Phe Thr
                85                  90                  95

Leu Asn Ile His Pro Val Glu Glu Glu Asp Val Ala Thr Tyr Tyr Cys
            100                 105                 110

Gln Gln Ser Tyr Glu Asp Pro Leu Thr Phe Gly Ala Gly Thr Lys Leu
        115                 120                 125

Glu Leu Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
            180                 185                 190

Lys Asp Ser Thr Tyr Ser Met Ser Ser Thr Leu Thr Leu Thr Lys Asp
        195                 200                 205

Glu Tyr Glu Arg His Asn Ser Tyr Thr Cys Glu Ala Thr His Lys Thr
    210                 215                 220

Ser Thr Ser Pro Ile Val Lys Ser Phe Asn Arg Asn Glu Cys
225                 230                 235

<210>  8
<211>  8
<212>  PRT
<213>  artificial

<220>
<223>  AntiMET-R heavy chain  - CDR-H1

<400>  8

Gly Tyr Thr Phe Thr Ser Tyr Trp
1               5

<210>  9
<211>  8
<212>  PRT
<213>  artificial

<220>
<223>  AntiMET-R heavy chain  -  CDR-H2

<400>  9

Ile Asn Pro Ser Ser Gly Arg Thr
1               5


<210>  10
<211>  5
<212>  PRT
<213>  artificial

<220>
<223>  Anti-MET-R heavy chain  - CDR-H3

<400>  10

Ala Ser Arg Gly Tyr
1               5


<210>  11
<211>  10
<212>  PRT
<213>  artificial

<220>
<223>  AntiMET-R light chain  -  CDR-L1

<400>  11

Gln Ser Val Asp Tyr Asp Gly Gly Ser Tyr
1               5                   10


<210>  12
<211>  3
<212>  PRT
<213>  artificial

<220>
<223>  AntiMET-R light chain  - CDR-L2

<400>  12

Ala Ala Ser
1


<210>  13
<211>  9
<212>  PRT
<213>  artificial

<220>
<223>  AntiMET-R light chain  - CDR-L3

<400>  13

Gln Gln Ser Tyr Glu Asp Pro Leu Thr
1               5

SEQUENCE LISTING

<110>  Metheresis Translational Research SA

<120>  Anti-Met monoclonal antibody, fragments and derivatives thereof
       for use in tumor diagnosis, corresponding compositions and kits

<130>  BEP9657-CF

<160>  13

<170>  PatentIn version 3.3

<210>  1
<211>  1386
<212>  DNA
<213>  artificial

<220>
<223>  AntiMET-R heavy chain

<400>  1
atgggatgga gctatatcat cctctttttg gtagcaacag ctacagatgg ccactcccag      60

gtccaactgc agcagcctgg gactgaactg gtgaagcctg gggcttcagt gaagctgtcc     120

tgcaaggctt ctggctacac cttcaccagt tactggatac actgggtgaa gcagaggcct     180

ggacaaggcc ttgagtggat tggagagatt aatcctagca gcggtcgtac taactacaac     240

gagaaattca agaacaaggt cacagtgact gtagacaaat cttccaccac agcctacatg     300

caactcagca acctgacatc tgaggactct gcggtctatt actgtgcaag taggggctac     360

tggggccaag gcaccactct cacagtctcc tcagccaaaa caacagcccc atcggtctat     420

ccactggccc ctgtgtgtgg aaatacaact ggctcctcgg tgactctagg atgcctggtc     480

aagggttatt tccctgagcc agtgaccttg acctggaact ctggatccct gtccagtggt     540

gtgcacacct tcccagctgt cctgcagtct gacctctaca ccctcagcag ctcagtgact     600

gtaacctcga gcacctggcc cagccagtcc atcacctgca atgtggccca cccggcaagc     660

agcaccaagg tggacaagaa aattgagccc agagggccca caatcaagcc ctgtcctcca     720

tgcaaatgcc cagcacctaa cctcttgggt ggaccatccg tcttcatctt ccctccaaag     780

atcaaggatg tactcatgat ctccctgagc cccatagtca catgtgtggt ggtggatgtg     840

agcgaggatg acccagatgt ccagatcagc tggtttgtga acaacgtgga agtacacaca     900

gctcagacac aaacccatag agaggattac aacagtactc tccgggtggt cagtgccctc     960

cccatccagc accaggactg gatgagtggc aaggagttca aatgcaaggt caacaacaaa    1020

gacctcccag cgcccatcga gagaaccatc tcaaaaccca aagggtcagt aagagctcca    1080

caggtatatg tcttgcctcc accagaagaa gagatgacta agaaacaggt cactctgacc    1140

tgcatggtca cagacttcat gcctgaagac atttacgtgg agtggaccaa caacgggaaa    1200

acagagctaa actacaagaa cactgaacca gtcctggact ctgatggttc ttacttcatg    1260

tacagcaagc tgagagtgga aaagaagaac tgggtggaaa gaaatagcta ctcctgttca    1320

gtggtccacg agggtctgca caatcaccac acgactaaga gcttctcccg gactccgggt    1380

aaatga                                                               1386

<210>  2
<211>  717
<212>  DNA
<213>  artificial

<220>
<223>  AntiMET-R light chain

<400>  2
atggagacag acacaatcct gctatgggtg ctgctgctct gggttccagg ctccactggt      60

gacattgtgc tgacccaatc tccagcttct ttggctgtgt ctctagggca gagggccacc     120

atctcctgca aggccagcca aagtgttgat tatgatggtg gtagttatat gagttggttc     180

caacagagac caggacagcc acccaaactc ctcatctctg ctgcatccaa tctagaatct     240

gggatcccag ccaggtttag tggcagtggc tctgggacag acttcaccct caatatccat     300

cctgtggagg aggaggatgt tgcaacctat tactgtcagc aaagttatga ggatccgctc     360

acgttcggtg ctgggaccaa gctggagctg aaacgggctg atgctgcacc aactgtatcc     420

atcttcccac catccagtga gcagttaaca tctggaggtg cctcagtcgt gtgcttcttg     480

aacaacttct accccaaaga catcaatgtc aagtggaaga ttgatggcag tgaacgacaa     540

aatggcgtcc tgaacagttg gactgatcag gacagcaaag acagcaccta cagcatgagc     600

agcaccctca cgttgaccaa ggacgagtat gaacgacata acagctatac ctgtgaggcc     660

actcacaaga catctacttc acccattgtc aagagcttca acaggaatga gtgttag        717


<210>  3
<211>  1473
<212>  DNA
<213>  artificial

<220>
<223>  Anti-MET-R Heavy chain with a tag sequence to the 3'

<400>  3
atgggatgga gctatatcat cctctttttg gtagcaacag ctacagatgg ccactcccag      60

gtccaactgc agcagcctgg gactgaactg gtgaagcctg gggcttcagt gaagctgtcc     120

tgcaaggctt ctggctacac cttcaccagt tactggatac actgggtgaa gcagaggcct     180

ggacaaggcc ttgagtggat tggagagatt aatcctagca gcggtcgtac taactacaac     240

gagaaattca agaacaaggt cacagtgact gtagacaaat cttccaccac agcctacatg     300

caactcagca acctgacatc tgaggactct gcggtctatt actgtgcaag taggggctac     360

tggggccaag gcaccactct cacagtctcc tcagccaaaa caacagcccc atcggtctat     420

ccactggccc ctgtgtgtgg aaatacaact ggctcctcgg tgactctagg atgcctggtc     480

aagggttatt tccctgagcc agtgaccttg acctggaact ctggatccct gtccagtggt     540

gtgcacacct tcccagctgt cctgcagtct gacctctaca ccctcagcag ctcagtgact     600

gtaacctcga gcacctggcc cagccagtcc atcacctgca atgtggccca cccggcaagc     660

agcaccaagg tggacaagaa aattgagccc agagggccca caatcaagcc ctgtcctcca     720

tgcaaatgcc cagcacctaa cctcttgggt ggaccatccg tcttcatctt ccctccaaag 780

atcaaggatg tactcatgat ctccctgagc cccatagtca catgtgtggt ggtggatgtg 840

agcgaggatg acccagatgt ccagatcagc tggtttgtga acaacgtgga agtacacaca 900

gctcagacac aaacccatag agaggattac aacagtactc tccgggtggt cagtgccctc 960

cccatccagc accaggactg gatgagtggc aaggagttca aatgcaaggt caacaacaaa 1020

gacctcccag cgcccatcga gagaaccatc tcaaaaccca aagggtcagt aagagctcca 1080

caggtatatg tcttgcctcc accagaagaa gagatgacta agaaacaggt cactctgacc 1140

tgcatggtca cagacttcat gcctgaagac atttacgtgg agtggaccaa caacgggaaa 1200

acagagctaa actacaagaa cactgaacca gtcctggact ctgatggttc ttacttcatg 1260

tacagcaagc tgagagtgga aaagaagaac tgggtggaaa gaaatagcta ctcctgttca 1320

gtggtccacg agggtctgca caatcaccac acgactaaga gcttctcccg gactccgggt 1380

aaagctagct ctgactacaa ggacgacgat gacaagagcg attacaaaga cgatgatgat 1440

aagctgcagc atcaccacca tcatcaccat tga 1473

<210> 4
<211> 9
<212> DNA
<213> artificial

<220>
<223> synthetic promoter - upstream

<400> 4
acctgggtt 9

<210> 5
<211> 13
<212> DNA
<213> artificial

<220>
<223> synthetic promoter - downstream

<400> 5
attggttggt tgg 13

<210> 6
<211> 461
<212> PRT
<213> artificial

<220>
<223> AntiMET-R heavy chain

<400> 6

Met Gly Trp Ser Tyr Ile Ile Leu Phe Leu Val Ala Thr Ala Thr Asp
1               5                   10                  15

Gly His Ser Gln Val Gln Leu Gln Gln Pro Gly Thr Glu Leu Val Lys
            20                  25                  30

Pro Gly Ala Ser Val Lys Leu Ser Cys Lys Ala Ser Gly Tyr Thr Phe
        35                  40                  45

Thr Ser Tyr Trp Ile His Trp Val Lys Gln Arg Pro Gly Gln Gly Leu
    50                  55                  60

Glu Trp Ile Gly Glu Ile Asn Pro Ser Ser Gly Arg Thr Asn Tyr Asn
65                  70                  75                  80

Glu Lys Phe Lys Asn Lys Val Thr Val Thr Val Asp Lys Ser Ser Thr
                85                  90                  95

Thr Ala Tyr Met Gln Leu Ser Asn Leu Thr Ser Glu Asp Ser Ala Val
            100                 105                 110

Tyr Tyr Cys Ala Ser Arg Gly Tyr Trp Gly Gln Gly Thr Thr Leu Thr
        115                 120                 125

Val Ser Ser Ala Lys Thr Thr Ala Pro Ser Val Tyr Pro Leu Ala Pro
    130                 135                 140

Val Cys Gly Asn Thr Thr Gly Ser Ser Val Thr Leu Gly Cys Leu Val
145                 150                 155                 160

Lys Gly Tyr Phe Pro Glu Pro Val Thr Leu Thr Trp Asn Ser Gly Ser
                165                 170                 175

Leu Ser Ser Gly Val His Thr Phe Pro Ala Val Leu Gln Ser Asp Leu
            180                 185                 190

Tyr Thr Leu Ser Ser Ser Val Thr Val Thr Ser Ser Thr Trp Pro Ser
        195                 200                 205

Gln Ser Ile Thr Cys Asn Val Ala His Pro Ala Ser Ser Thr Lys Val
    210                 215                 220

Asp Lys Lys Ile Glu Pro Arg Gly Pro Thr Ile Lys Pro Cys Pro Pro
225                 230                 235                 240

Cys Lys Cys Pro Ala Pro Asn Leu Leu Gly Gly Pro Ser Val Phe Ile
                245                 250                 255

Phe Pro Pro Lys Ile Lys Asp Val Leu Met Ile Ser Leu Ser Pro Ile
            260                 265                 270

Val Thr Cys Val Val Val Asp Val Ser Glu Asp Asp Pro Asp Val Gln
        275                 280                 285

Ile Ser Trp Phe Val Asn Asn Val Glu Val His Thr Ala Gln Thr Gln
    290                 295                 300

Thr His Arg Glu Asp Tyr Asn Ser Thr Leu Arg Val Val Ser Ala Leu

305                 310                 315                 320

Pro Ile Gln His Gln Asp Trp Met Ser Gly Lys Glu Phe Lys Cys Lys
                325                 330                 335

Val Asn Asn Lys Asp Leu Pro Ala Pro Ile Glu Arg Thr Ile Ser Lys
            340                 345                 350

Pro Lys Gly Ser Val Arg Ala Pro Gln Val Tyr Val Leu Pro Pro Pro
        355                 360                 365

Glu Glu Glu Met Thr Lys Lys Gln Val Thr Leu Thr Cys Met Val Thr
    370                 375                 380

Asp Phe Met Pro Glu Asp Ile Tyr Val Glu Trp Thr Asn Asn Gly Lys
385                 390                 395                 400

Thr Glu Leu Asn Tyr Lys Asn Thr Glu Pro Val Leu Asp Ser Asp Gly
                405                 410                 415

Ser Tyr Phe Met Tyr Ser Lys Leu Arg Val Glu Lys Lys Asn Trp Val
            420                 425                 430

Glu Arg Asn Ser Tyr Ser Cys Ser Val Val His Glu Gly Leu His Asn
        435                 440                 445

His His Thr Thr Lys Ser Phe Ser Arg Thr Pro Gly Lys
    450                 455                 460

<210>  7
<211>  238
<212>  PRT
<213>  artificial

<220>
<223>  AntiMET-R light chain

<400>  7

Met Glu Thr Asp Thr Ile Leu Leu Trp Val Leu Leu Leu Trp Val Pro
1               5                   10                  15

Gly Ser Thr Gly Asp Ile Val Leu Thr Gln Ser Pro Ala Ser Leu Ala
            20                  25                  30

Val Ser Leu Gly Gln Arg Ala Thr Ile Ser Cys Lys Ala Ser Gln Ser
        35                  40                  45

Val Asp Tyr Asp Gly Gly Ser Tyr Met Ser Trp Phe Gln Gln Arg Pro
    50                  55                  60

Gly Gln Pro Pro Lys Leu Leu Ile Ser Ala Ala Ser Asn Leu Glu Ser
65                  70                  75                  80

Gly Ile Pro Ala Arg Phe Ser Gly Ser Gly Ser Gly Thr Asp Phe Thr
                85                  90                  95

Leu Asn Ile His Pro Val Glu Glu Glu Asp Val Ala Thr Tyr Tyr Cys
            100                 105                 110

Gln Gln Ser Tyr Glu Asp Pro Leu Thr Phe Gly Ala Gly Thr Lys Leu
        115                 120                 125

Glu Leu Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
            180                 185                 190

Lys Asp Ser Thr Tyr Ser Met Ser Ser Thr Leu Thr Leu Thr Lys Asp
        195                 200                 205

Glu Tyr Glu Arg His Asn Ser Tyr Thr Cys Glu Ala Thr His Lys Thr
    210                 215                 220

Ser Thr Ser Pro Ile Val Lys Ser Phe Asn Arg Asn Glu Cys
225                 230                 235

<210>  8
<211>  8
<212>  PRT
<213>  artificial

<220>
<223>  AntiMET-R heavy chain  - CDR-H1

<400>  8

Gly Tyr Thr Phe Thr Ser Tyr Trp
1               5

<210>  9
<211>  8
<212>  PRT
<213>  artificial

<220>
<223>  AntiMET-R heavy chain  -  CDR-H2

<400>  9

Ile Asn Pro Ser Ser Gly Arg Thr
1               5

<210>  10

<211>  5
<212>  PRT
<213>  artificial

<220>
<223>  Anti-MET-R heavy chain  - CDR-H3

<400>  10

Ala Ser Arg Gly Tyr
1               5


<210>  11
<211>  10
<212>  PRT
<213>  artificial

<220>
<223>  AntiMET-R light chain  -  CDR-L1

<400>  11

Gln Ser Val Asp Tyr Asp Gly Gly Ser Tyr
1               5                   10


<210>  12
<211>  3
<212>  PRT
<213>  artificial

<220>
<223>  AntiMET-R light chain  - CDR-L2

<400>  12

Ala Ala Ser
1


<210>  13
<211>  9
<212>  PRT
<213>  artificial

<220>
<223>  AntiMET-R light chain  - CDR-L3

<400>  13

Gln Gln Ser Tyr Glu Asp Pro Leu Thr
1               5